Europäisches Patentamt

European Patent Office

Office européen des brevets

(19)

(11) Veröffentlichungsnummer: **0 231 725 B1**

## (12) EUROPÄISCHE PATENTSCHRIFT

(45) Veröffentlichungstag der Patentschrift: 23.10.91

(21) Anmeldenummer: 86810138.7

(22) Anmeldetag: 24.03.86

(51) Int. Cl.5: **A61M 29/02, A61M 25/00**

(54) **Katheter zur Behandlung von verengten Stellen, beispielsweise in einem Blutgefäss.**

(30) Priorität: 29.11.85 CH 5108/85

(43) Veröffentlichungstag der Anmeldung:
12.08.87 Patentblatt 87/33

(45) Bekanntmachung des Hinweises auf die
Patenterteilung:
23.10.91 Patentblatt 91/43

(84) Benannte Vertragsstaaten:
**AT BE CH DE FR GB IT LI LU NL SE**

(56) Entgegenhaltungen:
**WO-A-82/03557**
**WO-A-83/01893**
**CH-A- 616 337**
**DE-A- 2 823 192**

(73) Patentinhaber: **Schneider (Europe) AG**
**Schärenmoosstrasse 115**
**CH-8052 Zürich(CH)**

(72) Erfinder: **Hofmann, Eugen**
**Hardhof 14**
**CH-8064 Zürich(CH)**

(74) Vertreter: **White, William et al**
**Patentanwalts-Bureau Isler AG Postfach**
**6940**
**CH-8023 Zürich(CH)**

**Beschreibung**

Die Erfindung betrifft einen Katheter nach dem Oberbegriff des unabhängigen Patentanspruchs 1. Siehe, z.B., den in CH-A-616337 beschriebenen Katheter.

Katheter diser Gattung werden beispielsweise zur Behandlung von arteriosklerotischen Gefässverengungen seit einiger Zeit eingesetzt. Diese Katheter weisen am vorderen Ende einen ausdehnbaren Ballon auf, der in gefaltetem Zustand in den Bereich der Engstelle gebracht wird. Ueber einen von aussen zugänglichen Kanal wird der Ballon mit Flüssigkeit gefüllt, so dass sich dieser im Umfang ausdehnt und das die Verengung verursachende Gewebe nach aussen in die Gefässwand gedrückt wird.

Durch den ausgedehnten Ballon wird die Engstelle vollständig verschlossen, weshalb die Behandlung nach kurzer Zeitdauer unterbrochen werden muss, um beispielsweise eine distale Ischämie zu vermeiden.

Durch die erforderlichen Unterbrüche wird die gesamte Behandlungsdauer entsprechend verlängert.

Der Erfindung liegt die Aufgabe zugrunde, einen Katheter der eingangs genannten Gattung zu schaffen, bei der auch während einer länger dauernden Dehnung der Engstelle die Blut- und Sauerstoffversorgung der nachgeordneten Organe sichergestellt ist. Der Katheter soll kostengünstig herstellbar und trotzdem sicher sein.

Die Aufgabe wird mit einem Katheter mit den Merkmalen im Kennzeichen des unabhängigen Patentanspruchs 1 gelöst.

Im ausgedehnten Zustand bilden die Dilatationselemente und die Wand der Engstelle mehrere Kanäle, durch die Körperflüssigkeit oder ein Gas frei durchtreten können. So bilden sich bei drei Dilatationselementen und den Trägerschlauch herum und peripher zwischen der Wand der Engstelle und den Wandungen der Dilatationselemente je drei durchgehende und sich in Längsrichtung des Katheters erstreckende Kanäle. Ein Durchfluss der Körperflüssigkeit ist auch dann gewährleistet, wenn ein Teil der Kanäle verstopft ist.

Dadurch, dass anstelle eines grossen Dilatationselementes mehrere entsprechend kleinere Dilatationselemente verwendet werden, ist ein grösserer Dilatationsdruck möglich. Dies deshalb, weil kleinere Dilatationselemente mit einem grösseren Innendruck beaufschlagt werden können. So kann beispielsweise ein schlauchförmiges Dilatationselement mit einem Durchmesser von 18 mm lediglich mit einem Innendruck von $2 \cdot 10^5$ Pa beaufschlagt werden, während ein entsprechendes Dilatationselement mit einem Durchmesser von 8 mm einem Innendruck von $8 \cdot 10^5$ Pa standhält.

Verglichen mit den bekannten Kathetern dieser Gattung kann die gesamte Behandlungsdauer wesentlich verkürzt werden, da die Dehnung der Engstelle nicht unterbrochen werden muss.

Vorteilhafte Weiterbildungen des Erfindungsgegenstandes sind in den abhängigen Ansprüchen aufgeführt.

Ein Ausführungsbeispiel der Erfindung wird nachfolgend anhand der Zeichnung näher erläutert. Es zeigen:

Fig. 1
einen in stark vergrössertem Massstab dargestellten Katheter mit drei Dilatationselementen,

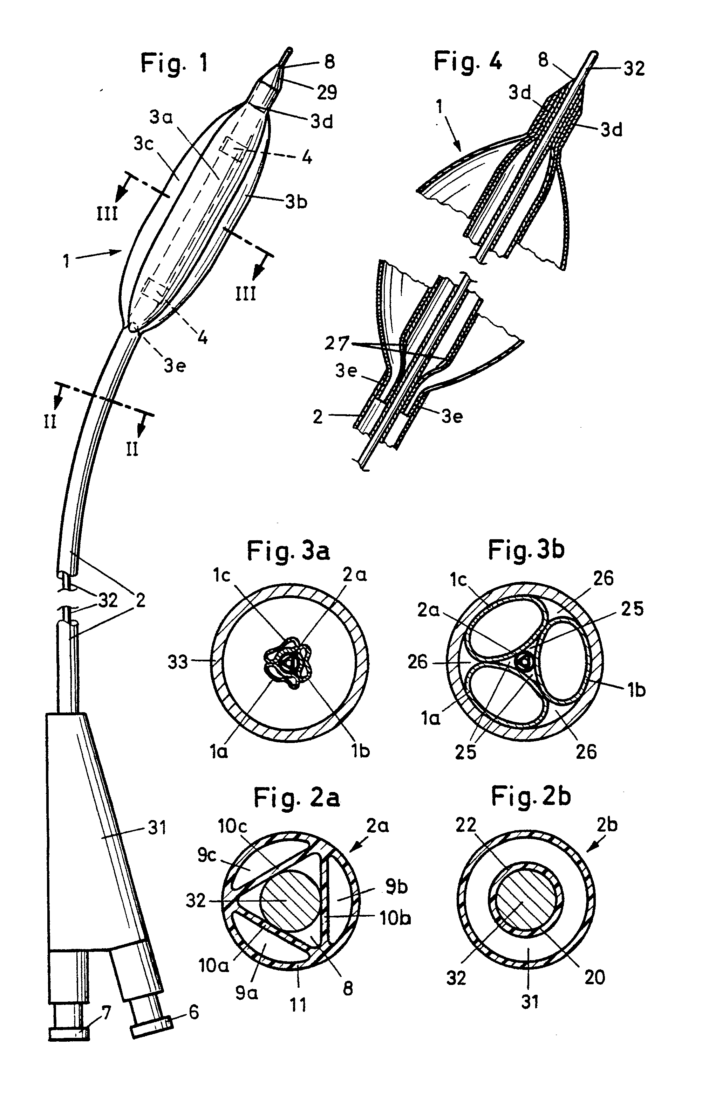

Fig. 2a und 2b
einen Querschnitt durch einen Trägerschlauch entland der Linie II-II der Fig. 1,

Fig. 3a
einen Querschnitt durch einen Dilatationsbereich mit gefalteten Wandungen und schematisch die Wandung einer Engstelle,

Fig. 3b
wie Fig. 3a, wobei jedoch die Dilatationselemente im Umfang ausgedehnt sind, und

Fig. 4
einen Längsschnitt durch den Dilatationsbereich.

Bei dem in Fig. 1 dargestellten Katheter weist der Dilatationsbereich 1 drei Dilatationselemente 3a-3c auf, die bündelartig um einen Trägerschlauch 2 angeordnet sind. Die Dilatationselemente 3a-3c sind jeweils an einem Ende 3d verschlossen und am andern Ende 3e offen. Die Enden 3d sind aussen am Trägerschlauch 2 befestigt, beispielsweise daran angeschweisst. Die offenen Enden 3e sind je in eine Oeffnung 27 des Trägerschlauches 2 gesteckt und darin und auf der Innenseite des Trägerschlauches 2 befestigt, beispielsweise mit einem Cyanoakrylat-Kleber, angeklebt (Fig. 4). Ausbildungen des Trägerschlauches 2 sind in den Fig. 2a und 2b dargestellt und werden weiter unten näher erläutert. Die Innenräume der Dilatationselemente 3a-3c sind über Kanäle 9a-9c (Fig. 2) oder einen gemeinsamen Ringkanal (Fig. 2b) mit einer hier nicht dargestellten, an sich bekannten Druck/Saugpumpe verbunden. Zwischen den Enden 3d und 3e der Dilatationselemente 3a-3c kann der Trägerschlauch 2 1-lumig oder gleich wie ausserhalb dieses Bereichs ausgebildet sein.

Die Dilatationselemente 3a-3c weisen im ausgedehnten Zustand eine im Querschnitt etwa kreisrunde Wandung auf, können jedoch je nach Anwendungsgebiet beispielsweise auch elliptisch ausgebildet sein. Das Aufblasen der Dilatationselemente 3a-3c kann in bekannter Weise durch ein flüssiges Medium oder ein Gas erfolgen. Dazu sind die Innenräume der Dilatationselemente 3a-3c vorzugsweise miteinander verbunden, so dass diese jeweils gleichzeitig ausgedehnt werden. Bei Verwendung des Trägerschlauches gemäss Fig. 2a sind dazu die Kanäle 9a-9c pumpenseitig zusammengeführt. Denkbar ist jedoch auch eine getrennte Bedienung der Dilatationselemente 3a-3c.

Der Trägerschlauch 2 weist einen an der Spitze 29 nach aussen führenden Kanal 8 bzw. 20 auf, durch den ein Führungs-Draht 32 geschoben oder Kontrastmittel sowie Medikamente durchgeleitet werden können. Auf dem Trägerschlauch 2 können Markierelemente 4 aus einem geeigneten Metall zur Röntgenlokalisierung des Dilatationsbereiches angebracht sein.

Der Trägerschlauch 2 ist an einem Zwischenstück 31 mit einem Anschluss 6 für die Druck/Saugpumpe und einem Mess- und Injektionsanschluss 7 angeschlossen.

Als besonders zweckmässig hat sich der Einsatz eines Trägerschlauches 2a gemäss Fig. 2a erwiesen. Dieser weist eine zylindrische Wand 11 auf, die innen durch Trennwände 10a bis 10c in einem dreieckigen inneren Kanal 8 und drei äussere Kanäle 9a bis 9c unterteilt ist. Die äusseren Kanäle 9a bis 9c führen zur Druck/Saugpumpe sowie zu den Dilatationselementen 3a bis 3c, während der innere Kanal 8 an der Spitze 29 nach aussen tritt. Der Trägerschlauch kann jedoch auch wie in Fig. 2b dargestellt, ausgebildet sein. Dieser 2-lumige Schlauch 2b weist einen äusseren Ringkanal 31 auf, der mit den Dilatationselementen 3a-3c verbunden ist. Eine koaxiale Innenwand 22 bildet einen inneren Kanal 20, durch den ein Führungsdraht 32 eingeschoben ist. Bei dieser Ausführung sind die Zwischenräume im Durchgang 31 zwischen den Enden 3e der Dilatationselemente mit Stücken aus einem geeigneten Siegelmaterial, wie beispielsweise einem Epoxypolymer, gefüllt (in der Zeichnung nicht dargestellt). Die Schläuche 2a und 2b sind flexibel und können beispielsweise durch Extrudieren aus einem geeigneten Kunststoff hergestellt werden.

Der erfindungsgemässe Katheter wird in an sich bekannter Weise über den Führungsdraht 32 in den Bereich der zu erweiternden Stelle gebracht, wobei die Dilatationselemente 3a-3c wie in Fig. 3a schematisch dargestellt, gefaltet sind.

Die Dilatationselemente 3a-3c werden nun durch Ueberdruck auf den erforderlichen Umfang dilatiert. In diesem Zustand sind die Wandungen 1a-1c der Dilatationselemente 3a-3c im Querschnitt etwa kreisförmig und drücken aussenseitig gegen die Wand 33 der Engstelle (Fig. 3b). Beim Aufblasen der Dilatationselemente 3a-3c bilden sich zentrale Kanäle 25 und periphere Kanäle 26, die in Längsrichtung des Katheters verlaufen und durchgehend sind, so dass durch diese mit wenig Widerstand Körperflüssigkeit hindurchtreten kann.

Da der Fluss der Körperflüssigkeit nicht unterbrochen ist, steht eine ausreichend lange Eingriffszeit zur Verfügung, während der die Dehnung der Engstelle nicht unterbrochen werden muss. Gegenüber den bekannten Kathetern kann dadurch die gesamte Behandlungsdauer wesentlich reduziert werden.

Der erfindungsgemässe Katheter kann zur Aufweitung von Engstellen bei allen Arten von Körperflüssigkeit führenden Gefässen eingesetzt werden. Weitere Anwendungsgebiete sind beispielsweise die Behandlung von Verengungen in der Speiseröhre oder im Gallengang. Gleichzeitig mit der Dehnung der Engstelle können durch den mittleren Kanal chemische Substanzen in den Körper eingebracht oder beispielsweise der Blutdruck gemessen werden.

Der erfindungsgemässe Katheter eignet sich insbesondere für valvuloplastische Verfahren, bei welchen die Flügel stenotisierter Herzklappen, insbesondere Pulmonal-, Aorta- und Mitralklappen, auf die Seite geschoben werden, um die Herzklappe wieder zu öffnen und ihre volle Funktionsfähigkeit wieder herzustellen. Dies kann ohne eine Operation am offenen Herz durchgeführt werden. Die Vorteile beim Gebrauch des erfindungsgemässen Katheters, im Vergleich zu einem Dilatationskatheter mit nur einem expandierbaren Dilatationselement, sind der erhöhte Flächendruck, der bei Verwendung von drei Dilatationselementen zum Oeffnen der Klappe ausgeübt werden kann und die Möglichkeit, diesen Druck, wie in Fig. 3b gezeigt, in Bereichen zu konzentrieren, die um $120°$ versetzt sind, was besonders geeignet ist zum Oeffnen der verkalkten Gefässe von stenotisierten dreiklappigen Herzklappen. In einem bevorzugten valvuloplastischen Verfahren zum Oeffnen einer stenotisierten Aortaklappe beispielsweise, wird ein erfindungsgemässer Katheter mit gefalteten und nicht expandierten Dilatationselementen auf einem geeigneten Führungsdraht 32 in die Femoralarterie eingeführt. Der Katheter und der Führungsdraht werden zusammen vorgeschoben, worauf der Führungsdraht selbst zur stenotisierten Klappe und durch die verbleibende Oeffnung davon vorgeschoben wird. Der erfindungsgemässe Katheter wird dann entlang des Führungsdrahtes vorgeschoben bis sich der Dilatationsbereich im Bereich der Klappenöffnung befindet, worauf dann die Dilatationselemente zum Oeffnen der Herzklappe expandiert werden. Nach dem Zusammenfalten der Dilatationselemente werden der erfindungsgemässe Katheter und der Führungsdraht aus dem Körper des Patienten entfernt.

**Patentansprüche**

1.   Katheter zum Dilatieren von verengten Stellen im kardiovaskulären System eines Patienten, mit einem Trägerschlauch (2), der an einem Ende einen Dilatationsbereich (1) mit einer wenigstens teilweise faltbaren Wandung (1a-1c) aufweist, welcher Dilatationsbereich (1) über ein Lumen (9a-9c;31) mittels einer am anderen Ende des Trägerschlauches (2) anzuschliessenden Druck/Saugpumpe im Umfang ausdehnbar ist, dadurch gekennzeichnet, dass der Dilatationsbereich (1) wenigstens zwei schlauchförmig ausgebildete, sich in Längsrichtung des Trägerschlauches (2) erstreckende und jeweils an ihren Enden an diesem befestigte Dilatationselemente (3a-3c) aufweist, die nebeneinander um den Trägerschlauch (2) herum angeordnet sind, und dass die Wandungen (1a-1c) der ausgedehnten Dilatationselemente (3a-3c) einen etwa kreisförmigen Querschnitt aufweisen, derart, dass zwischen den Dilatationselementen (3a-3c) Durchgänge (25,26) vorhanden sind, die einen Durchfluss von Blut ermöglichen.

2. Katheter nach Anspruch 1, dadurch gekennzeichnet, dass drei etwa gleich ausgebildete Dilatationselemente (3a-3c) vorgesehen sind.

3. Katheter nach Anspruch 2, dadurch gekennzeichnet, dass der Trägerschlauch (2) zwischen den Enden (3d, 3e) der Dilatationselemente (3a-3c) 1-lumig ist.

4. Katheter nach einem der Ansprüche 1 bis 3, dadurch gekennzeichnet, dass die Dilatationselemente (3a-3c) je an einem Ende (3d) geschlossen und am andern Ende (3e) mit einem im Trägerschlauch (2) verlaufenden Kanal (31; 9a-9c) verbunden sind.

5. Katheter nach einem der Ansprüche 1 bis 4, dadurch gekennzeichnet, dass die Innenräume der Dilatationselemente (3a-3c) miteinander wirkverbunden sind.

6. Katheter nach einem der Ansprüche 1 bis 5, dadurch gekennzeichnet, dass der Trägerschlauch (2a) für jedes Element (3a-3c) einen separaten Kanal (9a-9c) aufweist.

## Claims

1. Catheter for dilating constricted places in the cardiovascular system of a patient, with a carrier tube (2) which comprises at one end a dilation zone (1) with an at least partially foldable wall (1a-1c), which dilation zone (1) can be expanded in size by a lumen (9a-9c; 31) by means of a pressure/suction pump to be connected to the other end of the carrier tube (2), characterised in that the dilation zone (1) comprises at least two tubular dilation elements (3a-3c), which extend in the longitudinal direction of the carrier tube (2), are in each case fastened to the latter at their ends and are arranged next to one another around the carrier tube (2), and that the walls (1a-1c) of the expanded dilation elements (3a-3c) have an approximately circular cross section, such that there are passages (25, 26) between the dilation elements (3a-3c) through which blood can flow.

2. Catheter according to claim 1, characterised in that three dilation elements (3a-3c) of approximately the same form are provided.

3. Catheter according to claim 2, characterised in that the carrier tube (2) comprises a single lumen between the ends (3d, 3e) of the dilation elements (3a-3c).

4. Catheter according to one of claims 1 to 3, characterised in that the dilation elements (3a-3c) are each closed at one end (3d) and connected at the other end (3e) to a channel (31; 9a-9c) extending in the carrier tube (2).

5. Catheter according to one of claims 1 to 4, characterised in that the interior spaces of the dilation elements (3a-3c) are operatively connected together.

6. Catheter according to one of claims 1 to 5, characterised in that the carrier tube (2a) comprises a separate channel (9a-9c) for each element (3a-3c).

## Revendications

1. Cathéter pour la dilatation de constrictions dans le système cardiovasculaire d'un patient, comportant un tuyau porteur (2) qui présente sur une extrémité une zone de dilatation (1) avec une paroi repliable au moins partiellement (1a-1c), laquelle zone de dilatation (1) par l'intermédiaire d'un orifice (9a-9c, 31) peut être mise en expansion périphérique au moyen d'une pompe de pression/aspiration raccordable sur une autre extrémité du tuyau porteur (2), caractérisé en ce que la zone de dilatation (1) présente au moins deux éléments de dilatation (3a-3c) en forme de tuyau flexible qui s'étendent dans la direction longitudinale du tuyau porteur (2) et sont respectivement fixés sur celui-ci par leurs extrémités, éléments flexibles qui sont agencés en juxtaposition autour du tuyau porteur (2) et en ce que les parois (1a-1c) des éléments de dilatation en expansion (3a-3c) présentent une section transversale sensiblement circulaire de telle sorte qu'entre les éléments de dilatation (3a-3c) sont prévus des passages (25, 26) qui permettent l'écoulement du sang.

2. Cathéter selon la revendication 1, caractérisé en ce que sont prévus trois éléments de dilatation de conception sensiblement identique (3a-3c).

3. Cathéter selon la revendication 2, caractérisé en ce que le tuyau porteur (2) comporte une lumière ou orifice entre les extrémités (3d, 3e) des éléments de dilatation (3a-3c).

4. Cathéter selon l'une des revendications 1 à 3, caractérisé en ce que les éléments de dilatation (3a-3c) sont respectivement fermés sur une extrémité (3d) et sont raccordés sur l'autre extrémité (3e) à un canal (31, 9a-9c) s'étendant dans le tuyau porteur (2).

5. Cathéter selon l'une des revendications 1 à 4, caractérisé en ce que les espaces intérieurs des éléments de dilatation (3a-3c) sont reliés ensemble de façon opérante.

6. Cathéter selon l'une des revendications 1 à 5, caractérisé en ce que le tuyau porteur (2a) présente un canal séparé (9a-9c) pour chaque élément (3a-3c).

Fig. 1

Fig. 4

Fig. 3a

Fig. 3b

Fig. 2a

Fig. 2b